# EUROPEAN PATENT APPLICATION

(11) **EP 1 526 377 A1**
(43) Date of publication of application: **27.04.2005**
(21) Application number: 04024438.6
(22) Date of filing: 13.10.2004
(51) Int. Cl.: G01N 25/72

(54) **Method for detecting defects in substantially wood-like products, particularly panels and the like, and associated apparatus**

(30) Priority: 21.10.2003 IT mo20030284
(71) Applicant: Benedetti, Paolo, 41100 Modena (IT)
(72) Inventor: Benedetti, Paolo, 41100 Modena (IT)
(74) Representative: Modiano, Guido, Dr.-Ing.

(57) **Abstract**

A method for detecting defects in substantially wood-like products, particularly panels and the like, comprising a step for two-dimensional visualization of an irradiation measurement of at least one surface (S) of a substantially wood-like product (P) so as to obtain a thermogram that can be correlated to the visualized surface, and a step for comparing the resulting thermogram with at least one reference thermogram, the defects to be detected being located in the portions where the resulting thermogram differs from the reference thermogram.

## Description

The present invention relates to a method for detecting defects in substantially wood-like products, particularly panels and the like, and to the associated apparatus.

It is known that along the lines used to process essentially wood-like products there are control stations suitable to check the quality of the parts in transit along said lines.

In particular, along systems for manufacturing panels obtained by hot pressing loose wood-like materials, such as fibers, chips or flakes, there are apparatuses for detecting defects in said panels, which are generally arranged downstream of the continuous forming press and/or along the line for facing the formed panels.

The defects to be detected may be of various kinds and require dedicated devices to be detected.

The presence of surface stains or cracks can be detected, for example, by using optical devices such as TV cameras or the like.

Defects linked to lack of uniformity of the formed panels, caused for example by the inclusion of foreign objects, by uneven pressing or by the presence of cracks or air bubbles, are instead detected by means of devices that utilize the different absorption of electromagnetic radiation (X-rays) or of ultrasound by the panels at said defects.

In the first type of device, the image of the panels obtained by X-raying, reconstructed on a display device such as a monitor or the like, allows to reveal the presence of defects.

However, these devices are not free from drawbacks, which are mainly due to the danger entailed by X-rays for the health of assigned operators working in the vicinity of said devices, to the high installation and operating costs, and to the system complications that their use entails in panel manufacturing lines.

These apparatuses in fact require the provision of appropriate shielding suitable to reduce the risks for the health of the assigned operators and entail high costs due to the energy consumption of the units for generating and emitting X-rays.

Devices that utilize ultrasound are instead substantially constituted by a plurality of emitters functionally associated with corresponding receivers, arranged on opposite sides of a surface for transferring the panels being processed and distributed transversely with respect to the advancement direction of said surface.

The emitters emit respective ultrasound beams of predefined intensity; according to the intensity detected by the receivers, it is therefore possible to detect the presence of defects in the tested panels.

However, even this type of device has drawbacks, due first of all to the fact that they allow to perform only discrete detection and do not allow to monitor the entire surface of the panels, that they are rather bulky, require complicated auxiliary equipment, for example for supplying power to the ultrasound emitters and for cooling them, and have some operational limitations.

Ultrasound devices in fact cannot be used in environments at very high temperatures, and therefore are ill-suited for installation in the immediate vicinity of forming presses, which as is known heat the panels in order to allow the polymerization of the binding materials used to aggregate the loose wood-like material.

Moreover, the various types of device currently known and the corresponding operating methods do not allow direct control over the parameters that affect the quality level of the panels formed at the press, such as in particular the advancement rate of the panels in transit and therefore the time of transit through said press.

When the characteristics of the type of panel to be formed change, such speed is currently adjusted by trial and error, i.e., by setting a first speed value and comparing, by means of laboratory tests, the mechanical strength of the resulting panel and a reference value, so as to determine a new speed for which said comparison has to be repeated.

It is therefore evident that this calibration process requires a long time and entails the production of many rejects.

Moreover, the number of attempts by means of which the ideal stead-state advancement speed is determined depends significantly on the experience of the assigned operators.

The aim of the present invention is to eliminate the above-noted drawbacks of the background art, by providing a method for detecting defects in substantially wood-like products, particularly panels and the like, and an associated apparatus, which allow to detect the presence of imperfections of any kind, such as for example stains, cracks, air bubbles, inclusion of foreign objects or nonuniformities.

Within this aim, an object of the present invention is to provide a method and an apparatus that allow to perform real-time feedback control of the speed with which the panels advance along the forming lines.

Another object of the present invention is to provide a method and an apparatus that can be used in any temperature condition and particularly downstream of the continuous forming press.

Another object of the present invention is to provide an apparatus that does not entail onerous complications of the system in which it is used, is compact, and simple, relatively easy to provide in practice, safe in use, effective in operation, and has a relatively low cost.

This aim and these and other objects that will become better apparent hereinafter are achieved by the present method for detecting defects in substantially wood-like products, particularly panels and the like, characterized in that it comprises a step for two-dimensional visualization of an irradiation measurement of at least one surface of a substantially wood-like product so as to obtain a thermogram that can be correlated to the visualized surface and a step for comparing the resulting thermogram with at least one reference thermogram, the defects to be detected being located in the portions where the resulting thermogram differs from the reference thermogram.

The apparatus for performing this method is characterized in that it comprises at least one thermographic device, which is adapted to visualize at least one surface of said product in order to obtain a corresponding thermogram, and at least one between a device for visualizing the thermogram obtained by means of said thermographic device and a control and management unit for comparing the resulting thermogram with at least one reference thermogram, the visualization device and/or the unit being functionally associated with said thermographic device by way of corresponding interface means.

Further characteristics and advantages of the present invention will become better apparent from the following detailed description of some preferred but not exclusive embodiments of a method for detecting defects in substantially wood-like products, particularly panels and the like, and of an associated apparatus, illustrated by way of non-limiting example in the accompanying drawings, wherein:
Figure 1 is a schematic sectional view of a panel without defects;
Figure 2 is a schematic sectional view of a panel with defects;
Figure 3 is a partial schematic side view of a first embodiment of the apparatus according to the invention;
Figure 4 is a schematic perspective view of a second embodiment of the apparatus according to the invention.

With reference to the figures, the reference numeral 1 generally designates an apparatus for detecting defects in substantially wood-like products, particularly panels and the like.

The application of the detection method according to the invention is particularly advantageous for analyzing panels P obtained by pressing loose wood-like material, such as chips, fibers or flakes.

As it is known, the manufacturing cycle of these panels P provides for a pressing step, which is performed by using conventional continuous presses, which compact the mixture of loose wood-like material and binding materials and heat it in order to facilitate its aggregation.

This method comprises a step for the two-dimensional visualization of the irradiation measurement of at least one surface S of the panel P to be inspected, so as to obtain a thermogram that can be correlated to said surface.

The examined surface S is generally the upper face of the panel P, which is arranged horizontally.

As it is known, thermographic inspection allows to detect the temperature of a body by measuring the intensity of the infrared radiation emitted by said body, which is proportional to said temperature according to the well-known Stefan-Boltzmann law.

Moreover, a thermogram substantially consists of a sort of mapping of the body being inspected, illustrating by means of different colors different emission levels and therefore different temperatures of said body.

The method further comprises a step for comparing the resulting thermogram with at least one reference thermogram in order to determine whether the analyzed panel P is to be rejected or not.

Any defects that are present in the panel P are in fact located at the portions where the resulting thermogram differs from the reference thermogram.

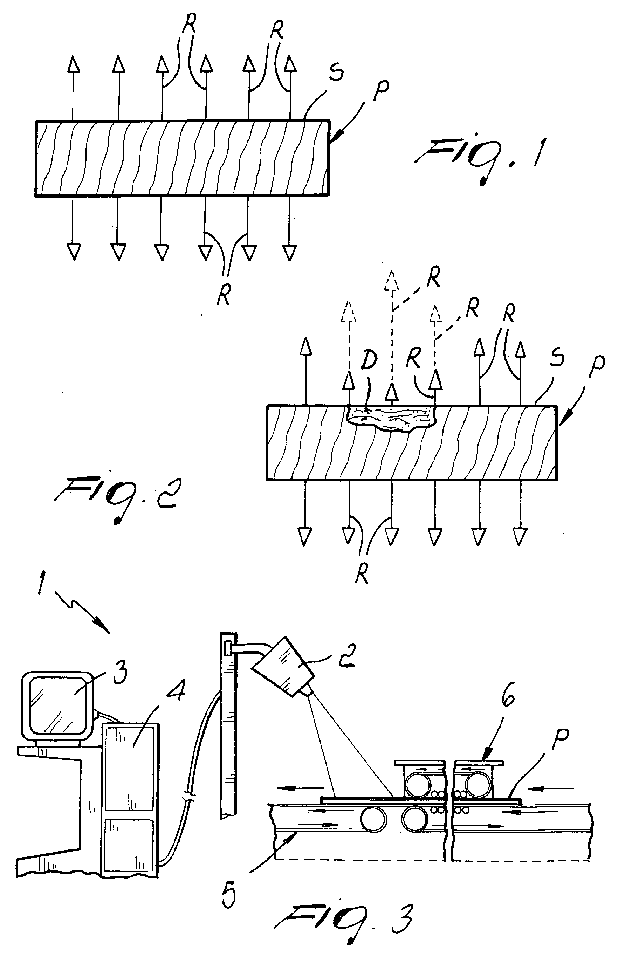

By way of example, Figure 1 illustrates a defect-free panel P: the emission of infrared radiation, represented schematically by the arrows R, is substantially uniform, and the thermogram obtained by inspecting this panel will be substantially identical to the reference thermogram, indicating a uniform temperature on the surface S.

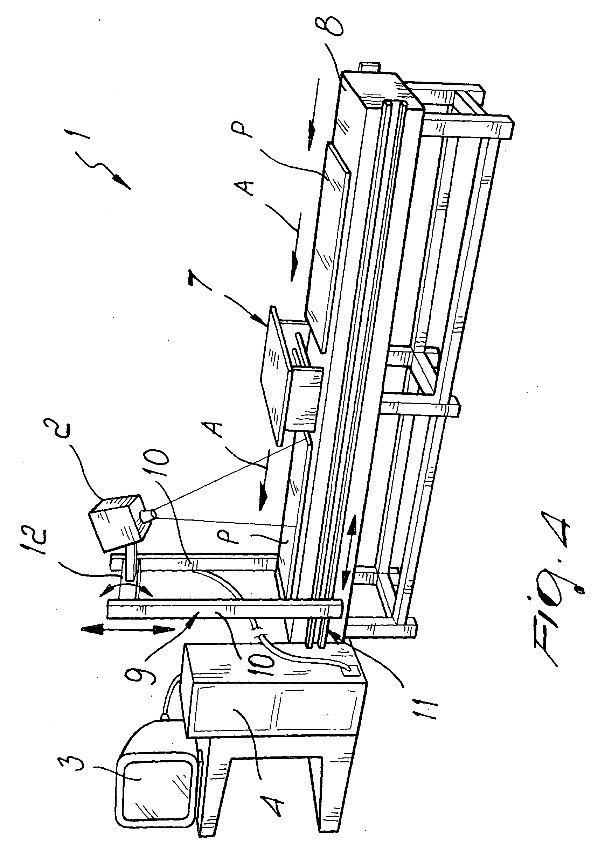

Figure 2 instead illustrates a panel P that has a defect D: the intensity of the radiation emitted by said panel is uneven, and in particular at the defect D there is a higher emission level (dashed arrows R) or a lower emission level (solid arrows R) than the remaining surface S, depending on the nature of said defect.

This type of analysis allows to reveal defects of various kinds, such as for example surface stains, cracks, air bubbles, inclusion of foreign and burst objects, which are currently detected by means of different operating methods depending on the type of defect being sought.

If the panel P to be checked is at a different temperature with respect to the environment, preferably a higher temperature, it can be sent directly to the visualization step by applying so-called passive thermography.

If instead the panel P to be analyzed is at ambient temperature, the method provides for a step for applying a thermal modification to said panel, performed upstream of the visualization step, which preferably consists in heating the panel P in a substantially uniform manner at a temperature that is higher than the ambient temperature.

In this last case, so-called active thermography is applied.

Finally, the method comprises a step for selecting the panel P, which is performed downstream of the comparison step.

The panel P is considered a reject if at least one portion of the corresponding resulting thermogram differs from the reference thermogram by more than a maximum temperature difference.

It should be noted that the method according to the invention allows to perform an inspection that is absolutely non-intrusive and allows to reveal defects of various kinds depending on the thermal and emissivity conditions of the panel P.

The apparatus 1 comprises at least one thermographic device that is sensitive to infrared radiation, such as a conventional thermal imaging camera 2 or the like, which is suitable to visualize the surface S and obtain its thermogram.

The apparatus 1 further comprises at least one between a device for visualizing the resulting thermogram, constituted for example by a monitor 3 or the like, and a management and control unit 4, for comparing the resulting thermogram with the reference thermogram.

The monitor 3 and/or the unit 4 are functionally associated with the thermal imaging camera 2 by way of corresponding interface means of a conventional type.

The unit 4 is programmed so as to perform a comparison between the resulting thermogram and the reference thermogram, in order to determine whether the panel P is to be rejected or not.

If the unit 4 detects that at least one portion of the resulting thermogram differs from the reference thermogram by more than a maximum temperature difference that is set in said unit, it considers the panel P as a reject.

Moreover, the unit 4 can produce a two-dimensional chart that illustrates, on the axis of the ordinates, the temperature of the panel P in a direction that is transverse to said panel and corresponds to the axis of the abscissas, which can then be displayed on the monitor 3.

As the position of the panel P with respect to said direction varies, said chart reveals, by means of a line, the temperature profile of the section of the panel P that in each instance is located at said direction.

The apparatus 1 may have means for indicating the detection of a panel P to be rejected, which are functionally associated with the unit 4.

Such indication means, not shown in the figures, may be constituted for example by acoustic or light-emitting warning means, by means for storing an identification code of the defective panel P, or by means for stopping the operation of the apparatus 1.

Conveniently, the use of the unit 4 allows to perform a quality control of the panels P without requiring the continuous presence of an operator next to the line; in this case, the monitor 3 is optional.

In an alternative embodiment, in which instead an operator is present next to the line, the apparatus 1 might not be provided with the unit 4; in this case, the operator stays in front of the monitor 3 and independently compares the resulting thermogram, displayed on said monitor, with the reference thermogram and determines whether the panel P is to be rejected or not.

In a first embodiment, shown in Figure 3, the apparatus 1 described above is applied at means for transferring the panels P along a processing line, such means being constituted for example by a continuous conveyor 5, which is arranged downstream of a continuous press 6 of the panel forming line.

In another embodiment, not shown, the apparatus 1 may be arranged for example at the means for transferring the panels P along a conventional facing line.

The panels P that exit from the press 6 are already at a higher temperature than the environment, for example on the order of 90-120° C, and therefore can be visualized directly by the thermal imaging camera 2.

Advantageously, this particular application of the apparatus 1 allows to perform real-time feedback time control of the efficiency of the press 6 and of the speed of the panels P in transit through that press.

By monitoring the temperature of the panels P that exit from the press 6 it is in fact possible to detect any malfunctions of said press (uneven pressing or heating), by comparing the resulting thermogram with one or more reference thermograms that correspond to previous production batches of good quality level, or detect a less than optimum transit speed of the panels P.

If, for example, the type of panels P to be formed is changed, the correct steady-state speed of transit through the press 6 can be determined rapidly, without producing an excessive amount of rejects and without requiring any laboratory tests, by monitoring the temperature of the panels P that exit from the press 6.

Moreover, by detecting the temperature of the panels P that exit from the press 6 it is possible to adjust temporarily the transit speed so as to contain significantly the production of rejects.

When a new forming line is installed, moreover, use of the apparatus 1 significantly reduces the setup times of said line.

If instead the apparatus 1 is used to inspect panels P at ambient temperature, means 7 for preheating said panels are provided upstream of the thermal imaging camera 2.

The preheating means 7 may provide, for example, at least one among an electric resistor, a heating lamp, a microwave generation device, or others.

Figure 4 illustrates a second embodiment, in which the apparatus 1 has a surface 8 for supporting the panels P, above which the thermal imaging camera 2 is arranged.

The surface 8 preferably can move along an advancement direction A and is constituted for example by a conventional conveyor belt or the like, not shown in detail.

The apparatus 1 has a frame 9 for supporting the thermal imaging camera 2, which comprises two uprights 10, which are arranged on opposite sides of the surface 8 and protrude above it and are associated so that they can move along the surface 8 by way of conventional guiding means 11 of the side-fitting type.

Conveniently, the frame 9 further comprises a beam 12, which is interposed between the uprights 10 and is associated so that it can slide along them toward and away from the surface 8, so that it is possible to adjust the level of the thermal imaging camera 2 with respect to said surface according to the characteristics and dimensions of the panels P to be monitored.

The thermal imaging camera 2 is preferably associated with the beam 12 so that it can oscillate.

In practice it has been found that the described invention achieves the intended aim and objects.

In particular, it should be noted that the method according to the invention can be performed by means of an apparatus that is compact and simple to install.

Moreover, the apparatus according to the invention performs the functions traditionally performed by a plurality of devices (optical visualization unit, X-ray devices and ultrasound devices), providing a total quality system for the products being processed.

The invention thus conceived is susceptible of numerous modifications and variations, all of which are within the scope of the appended claims.

All the details may further be replaced with other technically equivalent ones.

In practice, the materials used, as well as the shapes and dimensions, may be any according to requirements without thereby abandoning the scope of the protection of the appended claims.

The disclosures in Italian Patent Application No. M02003A000284 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for detecting defects in substantially wood-like products, particularly panels and the like, **characterized in that** it comprises a step for two-dimensional visualization of an irradiation measurement of at least one surface of a substantially wood-like product so as to obtain a thermogram that can be correlated to the visualized surface and a step for comparing the resulting thermogram with at least one reference thermogram, the defects to be detected being located in the portions where the resulting thermogram differs from the reference thermogram.

2. The method according to claim 1, comprising a step for applying a thermal modification to said product, which is performed before said visualization step.

3. The method according to one or more of the preceding claims, **characterized in that** said application step consists in heating said product in a substantially uniform manner.

4. The method according to one or more of the preceding claims, **characterized in that** it comprises a step for selecting said product, which is performed downstream of said comparison step, said product being a reject if at least one portion of the resulting thermogram differs from the reference thermogram by more than a maximum temperature difference.

5. An apparatus for performing the method according to one or more of the preceding claims, **characterized in that** it comprises at least one thermographic device, which is adapted to visualize at least one surface of said product in order to obtain a corresponding thermogram and at least one between a device for visualizing the thermogram obtained by means of said thermographic device and a management and control unit for comparing the resulting thermogram with at least one reference thermogram, the visualization device and/or the unit being functionally associated with said thermographic device by way of corresponding interface means.

6. The apparatus according to claim 5, **characterized in that** said thermographic device comprises at least one thermal imaging camera.

7. The apparatus according to one or more of the preceding claims, **characterized in that** said visualization device comprises at least one monitor or the like.

8. The apparatus according to one or more of the preceding claims, **characterized in that** it comprises means for preheating said product, which are arranged upstream of said thermographic device.

9. The apparatus according to one or more of the preceding claims, **characterized in that** said preheating means comprise at least one among an electric resistor, a heating lamp, a microwave generation device, or other devices.

10. The apparatus according to one or more of the preceding claims, **characterized in that** it comprises means for indicating the detection of a panel to be rejected, said means being functionally associated with said unit.

11. The apparatus according to one or more of the preceding claims, **characterized in that** it comprises a surface for supporting said product, above which said thermographic device is arranged.

12. The apparatus according to one or more of the preceding claims, **characterized in that** said supporting surface can move along a direction of advancement of said product.

13. The apparatus according to one or more of the preceding claims, **characterized in that** it comprises a frame for supporting said thermographic device.

14. The apparatus according to one or more of the preceding claims, **characterized in that** said frame is associated so that it can move along said supporting surface by way of the interposition of guiding means.

15. The apparatus according to one or more of the preceding claims, **characterized in that** said frame comprises a beam for supporting said visualization device, which can move toward and away from said supporting surface.

16. The apparatus according to one or more of the preceding claims, **characterized in that** said thermographic device is associated with said beam so that it can oscillate.

17. The apparatus according to one or more of the preceding claims, **characterized in that** said thermographic device is positioned above said means for transferring said product along a processing line.

18. Use of a thermographic device for detecting defects on substantially wood-like products, particularly panels and the like.
